# EUROPEAN PATENT SPECIFICATION

(11) **EP 1 850 774 B1**
(45) Date of publication and mention of the grant of the patent: **18.08.2010**
(21) Application number: 06727251.8
(22) Date of filing: 16.02.2006
(51) Int. Cl.: A61B 17/72

(54) **DISPOSABLE DEVICE FOR TREATMENT OF INFECTIONS OF HUMAN LIMBS**
EINWEG-VORRICHTUNG ZUR BEHANDLUNG VON INFEKTIONEN DER MENSCHLICHEN EXTREMITÄTEN
DISPOSITIF JETABLE DESTINE AU TRAITEMENT DES INFECTIONS DES MEMBRES HUMAINS

(30) Priority: 22.02.2005 IT VI20050049
(43) Date of publication of application: 07.11.2007
(73) Proprietor: Tecres S.P.A., 37066 Sommacampagna (VR) (IT)
(72) Inventor: SOFFIATTI, Renzo, I-37054 Nogara (IT); FACCIOLI, Giovanni, I-46040 Monzambano (IT)
(74) Representative: Feltrinelli, Secondo Andrea
(86) International application number: PCT/IB2006/000317
(87) International publication number: WO 2006/090226

(56) References cited:
- DE-A1- 19 605 735
- US-A- 4 863 444
- US-A- 5 571 193
- US-A- 5 618 286
- US-A1- 2002 147 451

## Description

### Field of the invention

The present invention is applicable in the technical field of surgical instruments, and particularly relates to a disposable device for treatment of infections of human limbs, more specifically limbs having long bones susceptible to stabilization by intramedullary nails.

### Background of the invention

Stabilization of long bones of the human body, following fractures, malformations or similar pathologic situations is known to be attained by using well-known devices such as intramedullary nails, consisting of more or less curved hollow or solid metal rods, which are fitted into the intramedullary cavity of the bones to be stabilized and are anchored to the stumps or parts to be stabilized by transverse-pins or screws, typically in the distal and proximal region of the limb. Once the fracture has been stabilized and fixed, the pins are removed and the nail is extracted from the medullary canal.

One problem associated to such treatment, in addition to the invasiveness of the implantation procedure, is the establishment of infection foci, which may locally develop due to the incompatibility of metal with the spongy medullary tissue and due to the bacteria that normally come in contact with the implantation site.

In order to minimize such drawbacks, it is highly recommended that the implantation of the above devices occurs in wholly sterile conditions, and that the relevant part is treated with substances adapted to prevent and/or treat such infections.

In an attempt to at least partly obviate the above drawbacks, solutions have been developed to allow *in situ* application of the above substances, alone or added to bone cement.

A solution is known in which a cover for the stabilizer device is made by using molds at the factory or directly at the surgical site immediately before implantation of such device.

This solution also has several apparent drawbacks.

First, it is a substantially manual solution, which requires special skills from the operator, who has to be properly trained therefor. Such an operation further involves many processing scraps, thereby causing a high material waste and an increase of the overall processing costs.

The thus covered device is also exposed for a sufficiently long time to air and to contact with foreign material, which involves a high risk of attack by bacteria and contaminants.

Furthermore, the covered device which comes out of the mold will exhibit many burrs along its longitudinal extension, which will have to be removed for proper positioning, thereby involving longer implantation times and consequent discomfort for the patient.

DE 19 605 735 A1 discloses a system comprising a tubular member which is made of a polymerised bone cement.

A further aspect of the invention relates to the need of temporarily replacing a nail for one of several different reasons, such as recidivation or dislocation of the implant, normally followed by surgical site infections.

In these situations, the existing nail has to be removed and the implant site has to be treated before insertion of the new nail. In the meantime, the geometry of the site shall be maintained unchanged, to prevent shortening and deformation of tissues, by providing a spacer device which can also prevent and/or treat the infections due to the old and new implant.

### Summary of the Invention

It is a main object of the present invention to overcome the above mentioned drawbacks by providing a device for infection treatment that exhibits high efficiency and cost-effectiveness.

A particular object is to provide a ready-to-use device for treatment of infections, which avoids the use of skilled personnel.

A further object is to provide a device for treatment of infections that allows to reduce the time of exposure thereof to the environment and assures highly sterile conditions.

Another object is to provide a device for treatment of infections, that allows an optimized use of materials.

Yet another object is to provide a device for treatment of infections, that allows fast removal of the cover.

These objects, as well as other objects that will be more apparent hereafter, are fulfilled by a disposable device according to claims 1 and 14.

This particular arrangement of the invention provides a ready-to-use device for treatment of infections, while avoiding the need of skilled personnel to fabricate it. Furthermore, this particular arrangement of the invention allows an optimized use of materials, and wholly eliminates processing scraps, while reducing the time required for fitting such cover onto the nail.

Preferably, the device for treatment of infections may be made of a material selected from the group including bone cements and reabsorbable or biodegradable bone cements. Thus, the device will be biologically compatible with the limb to be treated.

This tubular member may be suitably hollow, to allow fitting thereof onto an intramedullary nail.

Thanks to this arrangement, the device of the invention may be quickly removed from the nail, thereby allowing simple replacement of the latter after proper sterilization.

Suitably, the device of the invention may have connection means for anchoring of the tubular member to the intramedullary nail, which means preferably consist of an adhesive material, substantially uncured bone cement, designed to partly cover both the tubular member and the intramedullary nail on which it is fitted.

Advantageously, the device of the invention may be substantially continuous and have a predetermined length, corresponding to the distance between the distal and proximal holes of the intramedullary nail.

Thanks to this additional feature of the invention, the device may be coupled to the intramedullary nail in a still more stable and less invasive manner.

Preferably, the connection means may include a hole in the proximal or distal region of the tubular member. This will allow insertion of a screw for anchoring the limb to be treated to the intramedullary nail, to increase the stability of the treatment.

In another embodiment, the tubular member may be a strip of base material, which is wound in a substantially helical shape, wherein the turns may be equally spaced to define a through cavity, also having a substantially helical shape.

Thanks to this feature, the device of the invention allows an optimized use of the base material, and increases cost-effectiveness.

In accordance with a further aspect of the invention, there is provided an assembly for treatment of infections of human limbs according to claim 20, which comprises a sterile enclosure or blister, which is designed to contain a tubular member as described above.

This particular arrangement of the invention provides a device for treatment of infections which assures high sterility.

### Brief description of the drawings

Further features and advantages of the invention will be more apparent from the detailed description of a preferred, non-exclusive embodiment of a disposable device according to the invention, which is described by way of non-limiting example with the help of the annexed drawings, in which:

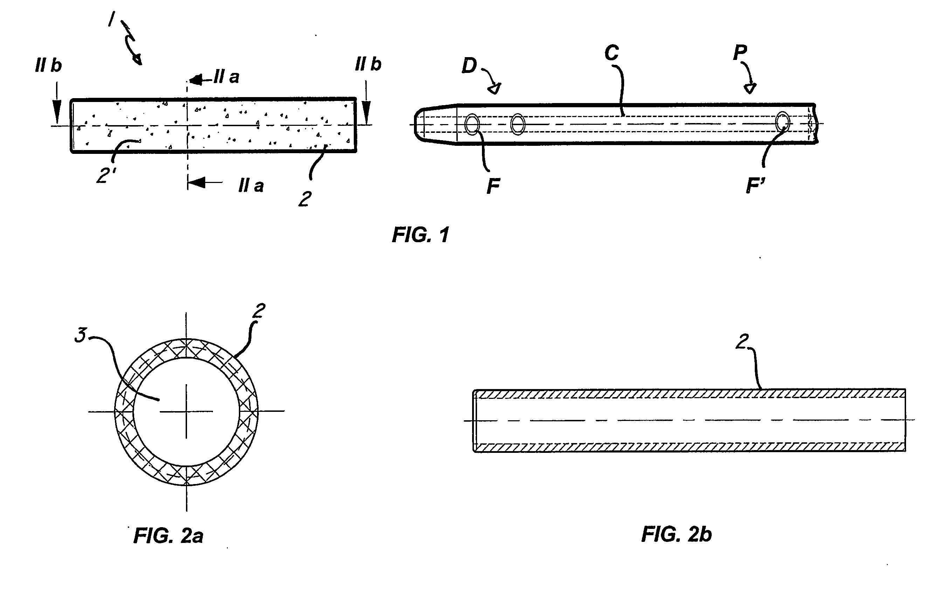

FIG. 1 is a sectioned view of one embodiment of the device according to the invention;
FIG. 2a is a broken away view of the device as shown in FIG. 1, taken along a plane *IIa-IIa;*
FIG. 2b is a sectioned view of the device as shown in FIG. 1, taken along a plane *IIb-IIb;*
FIG. 3 is a side view of a further embodiment of the device according to the invention;
FIG. 3a is a sectioned view of the device as shown in FIG. 3, taken along a plane *IIIa-IIIa;*
FIG. 3b is a sectioned view of the device as shown in FIG. 1, taken along a plane *IIIb-IIIb;*

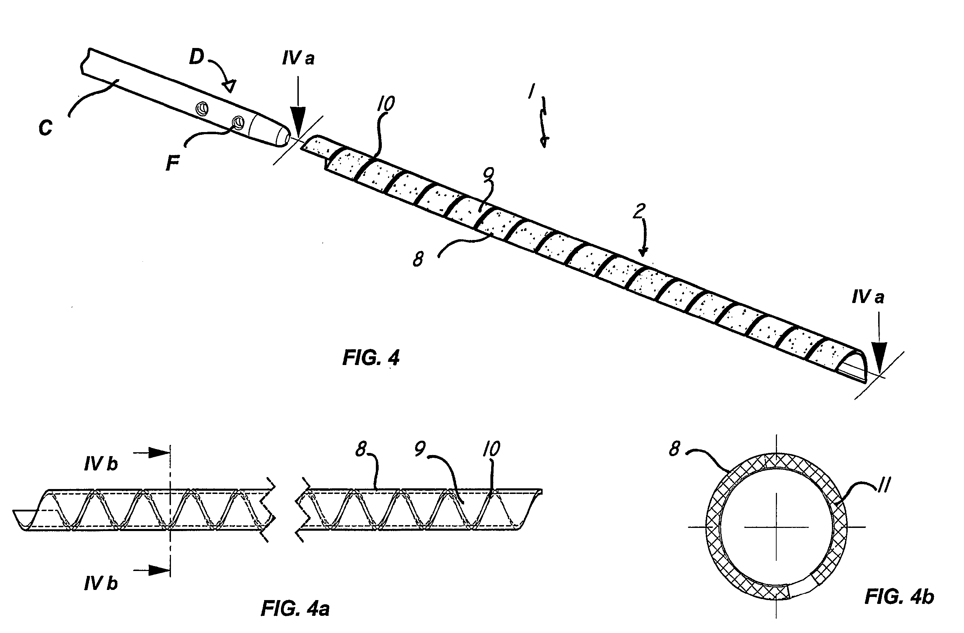

FIG. 4 is an axonometric view of another configuration of the device according to the invention;
FIG. 4a is a sectioned view of the device as shown in FIG. 4, taken along a plane *IVa-IVa;*
FIG. 4b is a sectioned view of the device as shown in FIG. 4, taken along a plane *IVb-IVb;*

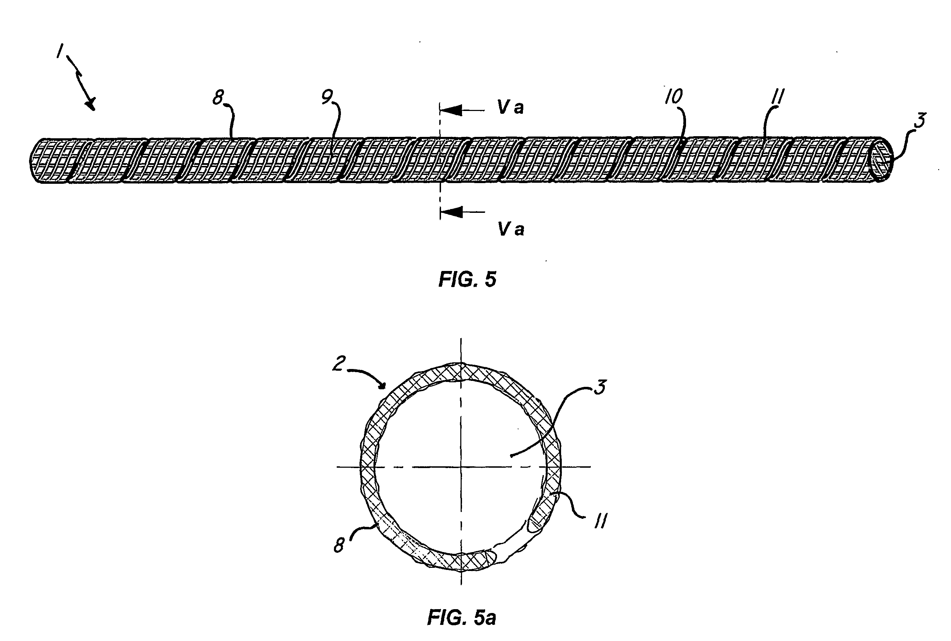

FIG. 5 is a side view of a further embodiment of the device of FIG. 4;
FIG. 5a is a sectioned view of the device as shown in FIG. 5, taken along a plane *Va-Va;*

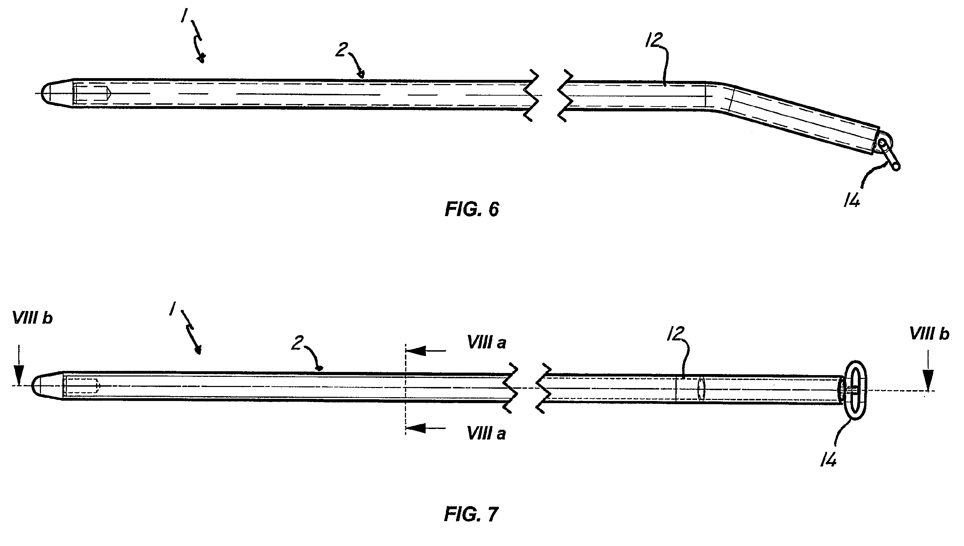

FIG. 6 is a side view of another configuration of the device according to the invention;
FIG. 7 is a further side view of the device of FIG. 6;

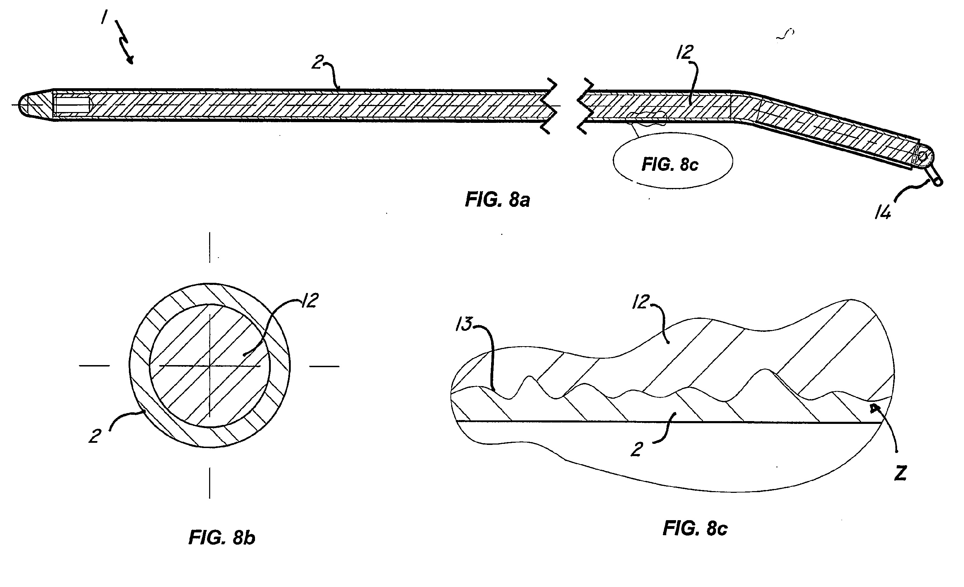

FIG. 8a is a sectioned view of the device as shown in FIG. 7, taken along a plane *VIIIb-VIIIb;*
FIG. 8b is a sectioned view of the device as shown in FIG. 7, taken along a plane *VIIIa-VIIIa;*
FIG. 8c is an enlarged view of a detail of FIG. 8a;

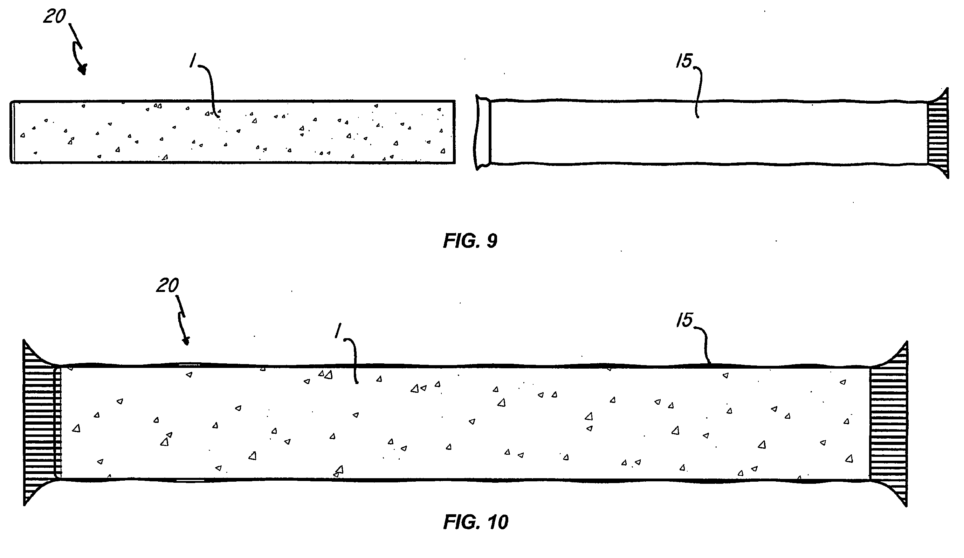

FIG. 9 is a side view of the assembly according to the invention;
FIG. 10 is a further side view of the assembly according to the invention.

### Detailed description of a preferred embodiment

Referring to the above figures, the device of the invention, generally denoted by numeral 1, is of the disposable type and will be particularly designed for stabilization of limbs having long bones by intramedullary nailing.

The device includes a tubular member 2 made of a relatively rigid and biologically compatible material, preferably selected from bone cements, which may also be of the reabsorbable or biodegradable type.

According to the invention, the tubular member 2 has pores 2' for impregnation with drugs or medicaments for treatment of infections, such as antibiotics, growth promoters, anti-inflammatory and/or amtitumor drugs, prior to or during insertion thereof in the stabilization site. In accordance with a first configuration of the invention, the tubular member 2 may have a central axial cavity 3 for the passage of an intramedullary nail C.

Furthermore, the tubular member 2 may include connection means 4 for anchorage thereof to the nail, which connections means preferably comprise an adhesive material 5 interacting with the member 2 and the intramedullary nail C on which it is fitted. Conveniently, the material 5 may be uncured bone cement, preferably laid or injected in at least one through cavity 6 of the member 2.

In accordance with a preferred embodiment of the invention, the tubular member 2 may be substantially continuous and have a predetermined length, preferably corresponding at least to the distance between the holes F and F' in the distal and proximal regions D, P respectively of the intramedullary nail C.

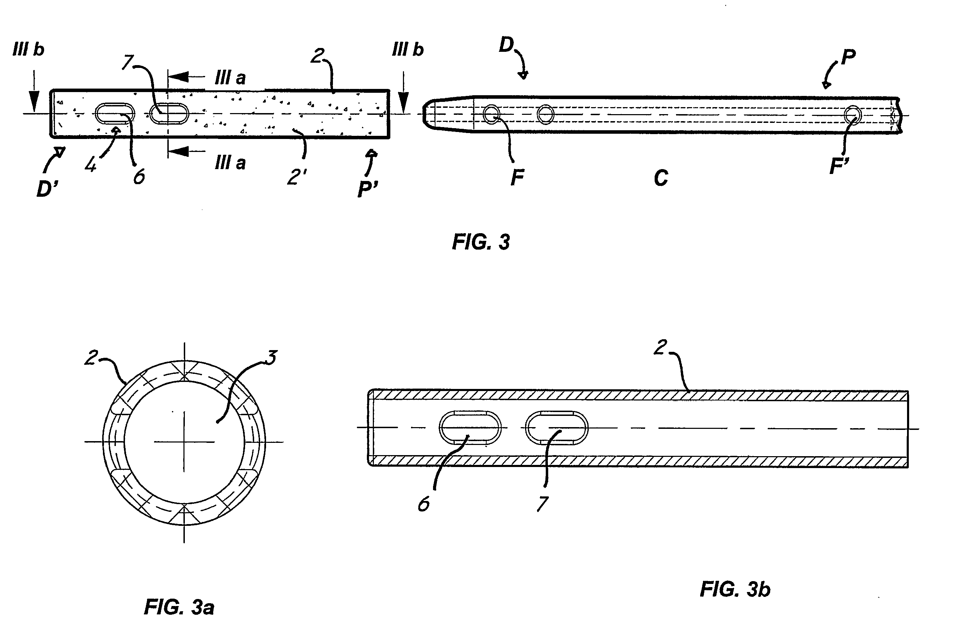

Advantageously, as particularly shown in FIG. 3, in addition to the cavity 6, a hole 7 may be provided in the proximal region P' or distal region D' of the member 2 for the passage of corresponding screws for anchorage of the intramedullary nail C, not shown in the figures. The screws will firmly secure the nail C, with the device 1 thereon, to the bone to be stabilized.

According to a further preferred, non exclusive embodiment of the invention, the member 2 may be a strip 8 of base material, which is wound in a substantially helical shape. Suitably, the turns 9 of such helical shape may be equally spaced, to define a through cavity 10, also having a substantially helical shape.

Preferably, the strip 8 may include an inner and/or outer reinforcement layer 11, made from a woven or non woven fibrous material, preferably of the mesh type. According to a further configuration of the invention, the bone cement member 2 may be provided with a stable reinforcement core 12, which is made of a relatively rigid metal or non-metal material, to actually define, as a whole, a spacer assembly.

Advantageously, the core 12 may have an irregular outer surface 13, defining an externally threaded anchorage area Z for the member 2.

Furthermore, a gripping member 14, preferably a ring, may be provided at the proximal end of the reinforcement core, to facilitate removal of the spacer assembly.

Here again the tubular member 2 may be impregnated with drugs or medicaments for treatment of infections, such as antibiotics, growth promoters, anti-inflammatory and/or antitumor drugs. Impregnation may occur prior to packaging of the device or upon insertion thereof on-site by the surgeon.

An assembly 20 for treatment of infections of human limbs, particularly limbs having long bones susceptible to stabilization by intramedullary nailing, comprises a sterile enclosure or blister 15 which is designed to contain the device 1, to assure the highest sterility and guarantee preservation conditions before installation.

This particular configuration of the invention provides a ready-to-use device for treatment of infections, which avoids the use of skilled personnel. The device further optimizes the use of materials, by considerably reducing processing scaps.

Finally, the construction of the above assembly provides a device for treatment of infections that allows to reduce the time of exposure thereof to the environment and assures highly sterile conditions.

The device of this invention is susceptible of a number of modifications and changes falling within the scope disclosed in the appended claims. All the details thereof may be replaced by other technically equivalent parts, and the materials may vary depending on different needs, without departure from the scope of the invention. While the device has been described with particular reference to the accompanying figures, the numerals referred to in the disclosure and claims are only used for the sake of a better intelligibility of the invention and shall not be intended to limit the claimed scope in any manner.

## Claims

1. A disposable device for treatment of infections of human limbs susceptible to stabilization by intramedullary nails, comprising a tubular member (2) made of a relatively rigid and biologically compatible base material, said tubular member (2) having pores (2') for impregnation with drugs or medicaments for treatment of infections, prior to or during insertion thereof in the stabilization site, and a central cavity (3) for the passage of said intramedullary nail (C), **characterized in that**
said device further comprises connection means (4) for anchorage of said tubular member (2) to said intramedullary nail (C).

2. A device as claimed in claim 1, **characterized in that** said base material is selected from he group including bone cements and reabsorbable or biodegradable bone cements.

3. A device as claimed in claim 1, **characterized in that** said tubular member (2) is substantially continuous and has a predetermined length.

4. A device as claimed in claim 3, **characterized in that** said predetermined length is at least equal to the distance between the holes (F, F") of the distal region (D) and the proximal region (P) of the intramedullary nail (C).

5. A device as claimed in claim 1, **characterized in that** said connection means (4) include an adhesive material (5) which is designed to interact with said tubular member (2) and the intramedullary nail (C) on which it is fitted.

6. A device as claimed in claim 5, **characterized in that** said adhesive material (5) is uncured bone cement.

7. A device as claimed in claim 6, **characterized in that** said uncured bone cement is introduced in at least one through cavity (6) of said tubular member (2).

8. A device as claimed in claim 1, **characterized in that** said convection means (4) include a hole (7) in the proximal region (P') or in the distal region (D') of said tubular member (2) for the passage of corresponding screws for anchorage to the intramedullary nail (C).

9. A device as claimed in claim 1 **characterized in that** said tubular member (2) is a strip of base material, which is wound in a substantially helical shape.

10. A device as claimed in claim 9, **characterized in that** the turns (9) of said substantially helical shape (8) are equally spaced, to define a through cavity (10), also having a substantially helical shape.

11. A device as claimed in claim 9, **characterized in that** said strip (8) of base material has an inner and/or outer reinforcement layer (11).

12. A device as claimed in claim 11, **characterized in** said reinforcement layer (11) is made of a woven or non-woven fibrous material.

13. A device as claimed in claim 11, **characterized in** said reinforcement layer (11) is made of a mesh material.

14. A device for treatment of infections of human limbs, comprising a tubular member (2) made of a relatively rigid and biologically compatible base material, said tubular member (2) having pores (2') which are suitable for impregnation with drugs or medicaments for treatment of infections, prior to or during insertion thereof in the stabilisation site, wherein said tubular member (2) is internally associated with a stable reinforcement core (12) made of a relatively rigid metal or non-metal material, **characterized** is that said device forms an intramedullary nail suitable for stabilisation of said human limbs.

15. A device as claimed in claim 14, **characterized in that** said reinforcement core (12) has an irregular outer surface (13), defining a permanent anchorage area (Z) for said tubular member (2).

16. A device as claimed in claim 15, **characterized in** said reinforcement layer (12) is externally threaded.

17. A device as claimed in claim 14, **characterized in** said reinforcement core (12) has a gripping member (14) to facilitate removal of the spacer assembly.

18. An assembly for treatment of infections of human limbs, particularly limbs having long bones susceptible to stabilization by intramedullary nailing, comprising a sterile packaging blister (15) which contains the device as claimed in any preceding claim.

## Patentansprüche

1. Einweg-Vorrichtung für die Behandlung von Infektionen von menschlichen Gliedmaßen, die durch intramedulläre Nägel stabilisiert werden können, umfassend ein röhrenförmiges Element (2), das aus einem relativ starren und biokompatiblen Grundmaterial besteht, wobei das röhrenförmige Element (2) Poren (2') zum Imprägnieren mit Arzneimitteln oder Medikamenten für die Behandlung von Infektionen vor oder während des Einsetzens desselben an der Stabilisierungsstelle und einen zentralen Hohlraum (3) für den Durchgang des intramedullären Nagels (C) aufweist; **dadurch gekennzeichnet, dass** die Vorrichtung ferner Verbindungsmittel (4) zum Verankern des röhrenförmigen Elements (2) am intramedullären Nagel (C) umfasst.

2. Vorrichtung nach Anspruch 1, **dadurch gekennzeichnet, dass** das Grundmaterial aus der Gruppe ausgewählt ist, die Knochenzemente und resorbierbare oder bioabbaubare Knochenzemente umfasst.

3. Vorrichtung nach Anspruch 1, **dadurch gekennzeichnet, dass** das röhrenförmige Element (2) im Wesentlichen ununterbrochen ist und eine vorbestimmte Länge hat.

4. Vorrichtung nach Anspruch 3, **dadurch gekennzeichnet, dass** die vorbestimmte Länge mindestens gleich dem Abstand zwischen den Löchern (F, F") des distalen Bereichs (D) und des proximalen Bereichs (P) des intramedullären Nagels (C) ist.

5. Vorrichtung nach Anspruch 1, **dadurch gekennzeichnet, dass** die Verbindungsmittel (4) ein Klebematerial (5) umfassen, das so gestaltet ist, dass es mit dem röhrenförmigen Element (2) und dem intramedullären Nagel (C), auf den es aufgesetzt wird, zusammenwirkt.

6. Vorrichtung nach Anspruch 5, **dadurch gekennzeichnet, dass** das Klebematerial (5) ungehärteter Knochenzement ist.

7. Vorrichtung nach Anspruch 6, **dadurch gekennzeichnet, dass** der ungehärtete Knochenzement in mindestens einen durchgehenden Hohlraum (6) des röhrenförmigen Elements (2) eingebracht wird.

8. Vorrichtung nach Anspruch 1, **dadurch gekennzeichnet, dass** die Verbindungsmittel (4) ein Loch (7) im proximalen Bereich (P') oder im distalen Bereich (D') des röhrenförmigen Elements (2) für den Durchgang von entsprechenden Schrauben für die Verankerung am intramedullären Nagel (C) umfassen.

9. Vorrichtung nach Anspruch 1, **dadurch gekennzeichnet, dass** das röhrenförmige Element (2) ein Streifen aus Grundmaterial ist, der im Wesentlichen spiralförmig gewunden ist.

10. Vorrichtung nach Anspruch 9, **dadurch gekennzeichnet, dass** die Windungen (9) der im Wesentlichen spiraligen Form (8) gleichmäßig beabstandet sind, um einen durchgehenden Hohlraum (10) zu definieren, der ebenfalls im Wesentlichen spiralförmig ist.

11. Vorrichtung nach Anspruch 9, **dadurch gekennzeichnet, dass** der Streifen (8) aus Grundmaterial eine innere und/oder äußere Verstärkungsschicht (11) aufweist.

12. Vorrichtung nach Anspruch 11, **dadurch gekennzeichnet, dass** die Verstärkungsschicht (11 ) aus einem gewebten oder nicht-gewebten Fasermaterial gesteht.

13. Vorrichtung nach Anspruch 11, **dadurch gekennzeichnet, dass** die Verstärkungsschicht (11 ) aus einem maschenartigen Material besteht.

14. Vorrichtung für die Behandlung von Infektionen von menschlichen Gliedmaßen, umfassend ein röhrenförmiges Element (2), das aus einem relativ starren und biokompatiblen Grundmaterial besteht, wobei das röhrenförmige Element (2) Poren (2') aufweist, die sich zum Imprägnieren mit Arzneimitteln oder Medikamenten für die Behandlung von Infektionen vor oder während des Einsetzens desselben an der Stabilisierungsstelle eignen, wobei das röhrenförmige Element (2) innen mit einem stabilen Verstärkungskern (12) verbunden ist, der aus einem relativ starren metallischen oder nichtmetallischen Material besteht, **dadurch gekennzeichnet, dass** die Vorrichtung einen intramedullären Nagel bildet, der sich zum Stabilisieren der besagten menschlichen Gliedmaßen eignet.

15. Vorrichtung nach Anspruch 14, **dadurch gekennzeichnet, dass** der Verstärkungskern (12) eine unregelmäßige äußere Oberfläche (13) aufweist, die eine Fläche (Z) für die dauerhafte Verankerung für das röhrenförmige Element (2) definiert.

16. Vorrichtung nach Anspruch 15, **dadurch gekennzeichnet, dass** die Verstärkungsschicht (12) außen ein Gewinde aufweist.

17. Vorrichtung nach Anspruch 14, **dadurch gekennzeichnet, dass** der Verstärkungskern (12) ein Greifelement (14) aufweist, um das Entfernen der Abstandhalteranordnung zu erleichtern.

18. Anordnung für die Behandlung von Infektionen von menschlichen Gliedmaßen, insbesondere von Gliedmaßen mit langen Knochen, die durch intramedulläres Nageln stabilisiert werden können, umfassend einen sterilen Verpackungsblister (15), der die Vorrichtung nach irgendeinem der vorstehenden Ansprüche enthält.

## Revendications

1. Dispositif jetable destiné au traitement des infections des membres humains, pouvant être stabilisés par des clous centromédullaire, comprenant un élément tubulaire (2) constitué d'un matériau de base relativement rigide et biologiquement compatible, ledit élément tubulaire (2) comportant des pores (2') permettant une imprégnation par des produits ou médicaments pour le traitement de l'infection, avant ou pendant l'insertion de l'élément dans le site de stabilisation, et une cavité centrale (3) pour le passage dudit clou centromédullaire (C),
**caractérisé en ce que** ledit dispositif comprend en outre des moyens de connexion (4) pour la fixation dudit élément tubulaire (2) audit clou centromédullaire (C).

2. Dispositif selon la revendication 1, **caractérisé en ce que** ledit matériau de base est sélectionné dans le groupe comprenant des ciments osseux et des ciments osseux résorbables et biodégradables.

3. Dispositif selon la revendication 1, **caractérisé en ce que** ledit élément tubulaire (2) est sensiblement continu et a une longueur prédéterminée.

4. Dispositif selon la revendication 3, **caractérisé en ce que** ladite longueur prédéterminée est au moins égale à la distance entre les trous (F, F") de la région distale (D) et la région proximale (P) du clou centromédullaire (C).

5. Dispositif selon la revendication 1, **caractérisé en ce que** lesdits moyens de connexion (4) comprennent un matériau adhésif (5) qui est conçu pour interagir avec ledit élément tubulaire (2) et le cou centromédullaire (C) sur lequel il est fixé.

6. Dispositif selon la revendication 5, **caractérisé en ce que** ledit matériau adhésif (5) est du ciment osseux non vulcanisé.

7. Dispositif selon la revendication 6, **caractérisé en ce que** ledit ciment osseux non vulcanisé est introduit dans au moins une cavité traversante (6) dudit élément tubulaire (2).

8. Dispositif selon la revendication 1, **caractérisé en ce que** lesdits moyens de connexion (4) comprennent un trou (7) dans la région distale (D') ou dans la région proximale (P') dudit élément tubulaire (2) pour le passage de vis correspondantes pour la fixation au clou centromédullaire (C).

9. Dispositif selon la revendication 1, **caractérisé en ce que** ledit élément tubulaire (2) est une bande de matériau de base, qui est enroulée avec un profil sensiblement hélicoïdal.

10. Dispositif selon la revendication 9, **caractérisé en ce que** les spires (9) dudit profil sensiblement hélicoïdal (8) sont uniformément espacées, pour définir une cavité traversante (10), présentant également un profil hélicoïdal.

11. Dispositif selon la revendication 9, **caractérisé en ce que** ladite bande (8) de matériau de base comporte une couche de renfort intérieure et/ou extérieure (11).

12. Dispositif selon la revendication 11, **caractérisé en ce que** ladite couche de renfort (11) est constituée d'une matière fibreuse tissée ou non tissée.

13. Dispositif selon la revendication 11, **caractérisé en ce que** ladite couche de renfort (11) est constituée d'un matériau à mailles.

14. Dispositif destiné au traitement des infections des membres humains, comprenant un élément tubulaire (2) constitué d'un matériau de base relativement rigide et biologiquement compatible, ledit élément tubulaire (2) comportant des pores (2') qui sont adaptés pour une imprégnation par des produits ou médicaments pour le traitement de l'infection, avant ou pendant l'insertion de l'élément dans le site de stabilisation, dans lequel ledit élément tubulaire (2) est associé de manière interne à une âme de renfort stable (12) constituée d'un matériau relativement rigide métallique ou non métallique, **caractérisé en ce que** ledit dispositif forme un clou centromédullaire adapté pour la stabilisation desdits membres humains.

15. Dispositif selon la revendication 14, **caractérisé en ce que** ladite âme de renfort (12) a une surface extérieure irrégulière (13), définissant une zone de fixation permanente (Z) dudit élément tubulaire (2).

16. Dispositif selon la revendication 15, **caractérisé en ce que** ladite couche de renfort (12) est filetée extérieurement.

17. Dispositif selon la revendication 14, **caractérisé en ce que** ladite âme de renfort (12) a un élément de prise (14) pour faciliter l'enlèvement de l'ensemble d'espacement.

18. Ensemble destiné au traitement des infections des membres humains, en particulier des membres constitués d'os long pouvant être stabilisés par enclouage centromédullaire, comprenant un emballage blister stérile (15) qui contient le dispositif selon l'une quelconque des revendications précédentes.
